# EUROPÄISCHE PATENTANMELDUNG

(11) **EP 2 090 581 A1**
(43) Veröffentlichungstag der Anmeldung: **19.08.2009**
(21) Anmeldenummer: 09150559.4
(22) Anmeldetag: 14.01.2009
(51) Int. Cl.: C07H 1/00, C07H 3/02

(54) **Verfahren zur Gewinnung von D-Galactose aus pflanzlichem Ausgangsmaterial durch saure Hydrolyse**

(30) Priorität: 18.01.2008 DE 102008000101
(71) Anmelder: Symrise GmbH & Co. KG, 37603 Holzminden (DE); Kaden Biochemicals GmbH, 22113 Hamburg (DE)
(72) Erfinder: Endlein, Dr. Edgar, 29559, Wrestedt (DE); Nagorny, Dr. Nicolai, 22949, Ammersbek (DE); Repenn, Florian, 21039 Hamburg (DE)
(74) Vertreter: Eisenführ, Speiser & Partner

(57) **Zusammenfassung**

Beschrieben wird ein Verfahren zur Gewinnung von D-Galactose, mit folgenden Schritten:
- Bereitstellen einer wässrigen Lösung oder Suspension von Galactoarabinan, wobei die wässrige Lösung bzw. Phase einen pH-Wert von kleiner oder gleich 3 aufweist,
- Erhitzen der Lösung oder Suspension auf eine Temperatur im Bereich von 80 bis 160 °C und Halten bei dieser Temperatur für einen Zeitraum im Bereich von 1 bis 40 Stunden,
- Abtrennen der gebildeten D-Galactose gegebenenfalls im Gemisch mit weiteren Stoffen aus der resultierenden Produktmischung.

## Beschreibung

Die vorliegende Erfindung betrifft ein Verfahren zur Gewinnung (und Aufreinigung) von Galactose, insbesondere D-Galactose ((+)-Galactose, CAS-Nr: 59-23-4), aus isoliertem pflanzlichem Ausgangsmaterial, insbesondere aus Arabinogalactan (INCl - Name: Galactoarabinan, CAS-Nr. 9036-66-2).

Galactose ist bedeutend für enzymatische und biochemische Untersuchungen, als Nährmedium sowie als Synthesebaustein. Galactose findet zudem weit verbreitete Anwendung im pharmazeutischen Sektor, sowohl als Trägerstoff als auch als Wirkstoff. D-Galactose hat insbesondere als Vorstufe für pharmazeutische Hilfsstoffe, als Wirkstoffträger oder auch als chiraler Baustein in der chemischen Synthese Bedeutung.

Verfahren zur synthetischen Herstellung von D-Galactose aus den entsprechenden Polyglycosiden durch Säurehydrolyse sind bereits bekannt.

Vielfach beschrieben ist die Gewinnung von D-Galactose mittels Hydrolyse von Lactose, siehe z.B. US 3,981,773, US 4,067748, EP 0 168 127 oder EP 0 499 164.

In bisherigen Verfahren, die Galactose im großtechnischen Maßstab aus Lactose darstellen, besteht immer die Problematik einer BSE-/TSE-Kontamination, da die verwendete Lactose von laktierenden Tieren stammt. Darüberhinaus sind Lactoseenthaltende Produkte oftmals nicht erwünscht, da diese für Menschen, die eine koschere Ernährung wünschen oder eine Lactose-Intoleranz haben, nicht geeignet sind.

Mit Galactose pflanzlichen Ursprungs ist es dagegen möglich, ein Produkt herzustellen, das nicht mit Allergenen, insbesondere nicht mit Lactose, kontaminiert ist.

Es besteht daher starkes Interesse an technischen Produktionsverfahren, bei denen die besagte Galactose in hoher Ausbeute und Reinheit aus pflanzlichem Ausgangsmaterial hergestellt werden kann.

In EP 1 046 719 wird hierzu die Hydrolyse ausgewählter oligosaccharidhaltiger Leguminosenzubereitungen vorgeschlagen, wie sie beispielsweise in Soja, Taps oder Sonnenblume vorkommen. Seltene Zucker, wie beispielsweise Arabinose, sollten dabei vorzugsweise nicht Bestandteil der zu hydrolysierenden Oligosaccharide gemäß EP 1 046 719 sein.

In Carbohydrate Research 2006 (341), 870-880 wird die nicht katalytische Freisetzung von Galactose aus Galactomannan bei Temperaturen von 180-240 °C und einer Reaktionszeit von 3-6 Minuten beschrieben. Galactoarabinan und dessen Hydrolyse werden nicht erwähnt.

In Carbohydrate Research 1997 (301) 89-93 wird die saure Hydrolyse von *Larix occidentalis* zur Bestimmung der Monosaccharidzusammensetzung beschrieben. Es erfolgt keine Angabe zu industriellen Prozessen.

In J. Inst. Chemists (India), Vol. 52, March, 1980, 57-58 und IN 158940 wird ein Prozess zur Darstellung von Galactose aus dem Gummi der grünen Frucht *Aegle marmelos* beschrieben. Dabei wird zunächst aus dem Gummi mittels milder Säurehydrolyse reines D-Galactan gewonnen, welches anschließend durch Hydrolyse in Gegenwart einer starken Säure in D-Galactose überführt wird. Bevorzugt werden dort Salz- und Schwefelsäure, wobei die maximale Ausbeute an D-Galactose mit Salzsäure (5 gew.%-ig) bei 25-30 % liegen soll, mit Schwefelsäure (4 gew.%-ig) hingegen wird eine Ausbeute von 50-55 % an D-Galactose erzielt. Nach Neutralisierung mit Bariumcarbonat wird dabei das Filtrat über einen sauren und einen basischen Ionentauscher filtriert und anschließend eingeengt. Der so erhaltene Sirup wird in Wasser aufgenommen und die D-Galactose unter Kühlung zur Kristallisation gebracht. Nach Papier-Chromatographie wird kristalline D-Galactose in einer Reinheit von 99,5 % erhalten. Die Herstellung erfolgt im Labormaßstab.

In J. Chem. Soc. 1948, 774 wird ε-Galactan aus *Larix decidua* und dessen Hydrolyse sowie Methylierung beschrieben. Zunächst wurde das Polysaccharid mit 0,01 N Schwefelsäure für 40 h bei 90°C erhitzt, die Lösung mit Bariumcarbonat neutralisiert und filtriert. Das Filtrat wurde zu einem Sirup eingeengt, welcher aus wässriger Lösung durch Zugabe von Alkohol fraktioniert wurde. Die dadurch erhaltene zweite Fraktion wurde 4 h lang mit siedender N-Schwefelsäure weiter hydrolysiert, diese Lösung ebenfalls mit Bariumcarbonat neutralisiert, filtriert und zu einem Sirup eingeengt, welcher kristallisierte. Durch Zerreiben mit Alkohol wurde kristalline D-Galactose erhalten.

In J. Chem. Soc. 1953, 1672 wird die Hydrolyse von ε-Galactan aus Lärche mit kalter oder heißer Salzsäure, gefolgt von einer Filtration und der Neutralisierung an basischem Ionentauscher, beschrieben. Dabei wurde das Filtrat eingeengt und in Alkohol eingetragen, woraufhin das Filtrat vom Niederschlag abgetrennt und zu einem Sirup eingeengt wurde. Der so erhaltene Sirup wurde über eine Säule aus Cellulose mit einer Mischung aus n-Butanol und Wasser fraktioniert, wobei zuerst L-Arabinose und anschließend D-Galactose eluierte.

In Chemistry and Industry 1952, 954 wird beschrieben, dass ε-Galactan der Lärche mittels Salzsäure hydrolysiert werden kann, wobei die Hydrolyse mit N-Salzsäure bei 20°C oder mit 0,01 N Salzsäure bei 100°C erfolgen kann. Die auf diese Weise erhaltene Saccharidmischung wurde an Cellulose fraktioniert, wobei Disaccharide aus L-Arabinose-Einheiten isoliert wurden.

In US 1,718,837 wird beschrieben, dass die Kohlenhydrate der Lärche *Larix occidentalis* mittels Oxidation mit Salpetersäure in Schleimsäure (englisch: mucic acid) umgesetzt werden können. Die thermische Behandlung eines wässrigen Holzextraktes oder des Holzes selbst in Gegenwart einer geringen Menge einer Mineralsäure führte zur Unlöslichkeit der im Extrakt vorhandenen Tannine, wobei das Galactan des Holzes zu einem gewissen Anteil zu Galactose hydrolysiert wird und die Galactose dadurch als Ausgangsmaterial für die Bildung von Schleimsäure gemäß US 1,718,837 dient.

WO 2005/001145 betrifft die Gewinnung von D-Galactose aus Pflanzenmaterial, wobei dort ein chromatographischer Schritt an einem Ionentauscher als wesentlich angesehen wird, um eine Fraktion zu erhalten, aus der anschließend die D-Galactose mittels Kristallisation gewonnen wird. Galactoarabinan und dessen Hydrolyse werden in WO 2005/001145 nicht erwähnt.

In GB 2 407 573 und WO 2005/042788 wird die Gewinnung von Arabinose aus pflanzlichen Fasern beschrieben, wobei als bevorzugte Pflanzenmaterialien Gummi Arabicum, Ghatti Gummi und Traganth angegeben werden.

Trotz der vorstehend genannten sowie weiterer bekannter Verfahren zur Freisetzung bzw. Gewinnung und Aufreinigung von D-Galactose aus pflanzlichem Material besteht weiterhin ein Bedarf an einem verbesserten Verfahren, welches insbesondere im industriellen Maßstab durchführbar sein sollte. Sämtliche bisher bekannten Verfahren besitzen jeweils wenigstens einen oder mehrere der nachfolgenden Nachteile:
geringe Produktivität (geringe Eduktkonzentration, niedrige Ausbeute, lange Reaktionszeit), geringe Reinheit, Entstehung unerwünschter Nebenprodukte (z.B. Zersetzungsprodukte aus der Pflanzenmatrix).

Es war daher eine primäre Aufgabe der vorliegenden Erfindung, ein Verfahren zur Gewinnung von D-Galactose aus pflanzlichem Ausgangsmaterial anzugeben, welches den Einsatz hoher Eduktkonzentrationen ermöglicht und zugleich zu hohen Produktausbeuten führt. Vorzugsweise sollte es in dem anzugebenden Verfahren möglich sein, D-Galactose in hoher Reinheit und möglichst frei von anorganischen Verunreinigungen zu gewinnen, wobei bevorzugt allenfalls ein sehr geringer Anteil an anderen Zuckern vorhanden sein sollte.

Die gestellte Aufgabe wird gelöst durch ein Verfahren zur Gewinnung von D-Galactose, mit folgenden Schritten:
- Bereitstellen einer wässrigen Lösung oder Suspension von Galactoarabinan, wobei die wässrige Lösung bzw. Phase (der Suspension) einen pH-Wert von kleiner oder gleich 3 aufweist,
- Erhitzen der Lösung bzw. Suspension auf eine Temperatur im Bereich von 80 bis 160 °C und Halten bei dieser Temperatur für einen Zeitraum im Bereich von 1 bis 40 Stunden (saure Hydrolyse),
- Abtrennen der gebildeten D-Galactose gegebenenfalls im Gemisch mit weiteren Stoffen aus der resultierenden Produktmischung.

Die vorliegende Erfindung basiert auf umfangreichen eigenen Untersuchungen zur hydrolytischen Gewinnung von D-Galactose aus natürlichen Bioploymeren. Die eigenen Untersuchungen haben gezeigt, dass bei den unterschiedlichen Biopolymeren die jeweils erhaltenen Hydrolysate in der Regel nicht geeignet waren, um D-Galactose im technischen bzw. industriellen Maßstab zu erhalten.

Es zeigte sich aber schließlich auch, dass das erfindungsgemäße Verfahren zur Gewinnung von D-Galactose aus Galactoarabinan (durch saure Hydrolyse) in besonderer Weise geeignet ist, die gestellte Aufgabe zu lösen.

Die wässrige Lösung oder Suspension von Galactoarabinan wird in einem erfindungsgemäßen Verfahren zur Gewinnung von D-Galactose vorzugsweise durch folgende Schritte bereitgestellt:
(i) Bereitstellen von Wasser,
(ii) Lösen oder Suspendieren von Galactoarabinan in dem in Schritt (i) bereitgestellten Wasser oder einem gemäß Schritt (iii) angesäuerten Wasser,
(iii) Zugabe einer starken anorganischen Säure (vorzugsweise Salzsäure, siehe unten) zu der gemäß Schritt (ii) erhaltenen Lösung oder Suspension oder zu dem in Schritt (i) bereitgestellten Wasser,
so dass die wässrige Phase der angesäuerten Lösung oder Suspension einen pH-Wert von kleiner oder gleich 3, vorzugsweise kleiner oder gleich 2 aufweist.

Die in einem erfindungsgemäßen Verfahren bereitgestellte wässrige Lösung bzw. Suspension von Galactoarabinan, wird demnach gemäß einer ersten Alternative hergestellt, indem Galactoarabinan in Wasser gelöst oder suspendiert wird und anschließend eine starke anorganische Säure zu dieser Lösung oder Suspension zugegeben wird. Vorzugsweise weist die somit angesäuerte Lösung bzw. Suspension einen pH-Wert von kleiner oder gleich 2 auf. Gemäß einer zweiten Alternative wird die oben beschriebene wässrige Lösung oder Suspension von Galactoarabinan hergestellt, indem eine starke anorganische Säure zu einem bereitgestellten Wasser hinzugegeben wird, so dass das angesäuerte Wasser einen pH-Wert von kleiner oder gleich 3, vorzugsweise kleiner oder gleich 2 aufweist, und danach Galactoarabinan in dem angesäuerten Wasser gelöst oder suspendiert wird.

Erfindungsgemäß bevorzugt wird Galactoarabinan aus der Lärche (Larix) eingesetzt, ein natürlich vorkommendes Polymer, welches Galactose und Arabinose enthält. Besonders geeignete Lärchenarten sind *Larix occidentalis, Larix larcicina, Larix lyallii, Larix decidua, Larix dahurica, Larix leptolepis,* und *Larix siberica.* Dementsprechend kann das erfindungsgemäß einzusetzende Galactoarabinan durch weitere aus der Lärche stammende Bestandteile verunreinigt sein.

In einer bevorzugten Ausführungsform des erfindungsgemäßen Verfahrens weist das Galactoarabinan ein mittleres Molekulargewicht im Bereich von 2000 - 250000 Dalton auf, bevorzugt ein mittleres Molekulargewicht im Bereich von 5000 - 100000 Dalton und besonders bevorzugt ein mittleres Molekulargewicht im Bereich von 5000 - 50000 Dalton.

Kommerziell erhältliche Galactoarabinane, die in einem erfindungsgemäßen Verfahren eingesetzt werden können, sind unter anderem unter den Marken- oder Produktnamen Larex^{®} UF, Lara Care^{®} A 200, Cleartrac oder FiberAid^{®} erhältlich, beispielsweise von Larex International oder Lonza Group.

Das erfindungsgemäß ausgewählte Startmaterial (z.B. Galactoarabinan) und die erfindungsgemäß gewählte Reaktionsführung (insbesondere: pH; Temperatur; Reaktionsdauer; eingesetzte Säure und pKₐ der eingesetzten Säure) ermöglichen ein industriell durchführbares Verfahren, in welchem eine gut kristallisierende D-Galactose in hoher Reinheit erhalten wird. Eine besonders bevorzugte Ausgestaltung stellt ein erfindungsgemäßes Verfahren (wie oben beschrieben) dar, in dem die bereitgestellte wässrige Lösung bzw. Suspension von Galactoarabinan 1 bis 35 Gew.-%, bevorzugt 5 bis 25 Gew.-%, Galactoarabinan enthält, bezogen auf das Gesamtgewicht der Lösung oder Suspension.

Besonders bevorzugt umfasst ein erfindungsgemäßes Verfahren, insbesondere in einer seiner bevorzugten Ausgestaltungen, den folgendem Schritt:
- Erhitzen der bereitgestellten Lösung oder Suspension auf eine Temperatur im Bereich von 90 bis 125 °C und Halten bei dieser Temperatur für einen Zeitraum im Bereich von 10 bis 30 Stunden.

Zudem weist ein erfindungsgemäßes Verfahren, vorzugsweise in einer vorstehend als bevorzugt bezeichneten Ausgestaltung, den folgenden Schritt auf:
- Neutralisation der nach dem Erhitzen und dem Halten der Temperatur vorliegenden Lösung oder Suspension, vorzugsweise an einem oder nacheinander an mehreren Ionenaustauschern.

Vorzugsweise wird demnach die wässrige Lösung bzw. Phase an einem Ionentauscher oder nacheinander an mehren Ionenaustauschers neutralisiert. Vorzugsweise erfolgt die Neutralisation nacheinander an einem basischen und einem sauren Ionentauscher oder nacheinander an einem basischen, einem sauren und einem weiteren basischen Ionentauscher.

Vorzugsweise erfolgt in einem erfindungsgemäßen Verfahren das Abtrennen der gebildeten D-Galactose (gegebenenfalls im Gemisch mit weiteren Stoffen) aus der resultierenden Produktmischung durch Kristallisation. Dementsprechend sind erfindungsgemäße Verfahren besonders bevorzugt, welche den folgenden Schritt umfassen:
- Abtrennen der gebildeten D-Galactose, gegebenenfalls im Gemisch mit weiteren Stoffen, aus der resultierenden Produktmischung durch Kristallisation.

Besonders bevorzugt ist eine Ausführung eines erfindungsgemäßen Verfahren mit folgenden Schritten:
- Bereitstellung der wässrigen Lösung oder Suspension von Galactoarabinan durch:
   (i) Bereitstellen von Wasser,
   (ii) Lösen oder Suspendieren von Galactoarabinan in dem in Schritt (i) bereitgestellten Wasser oder einem gemäß Schritt (iii) angesäuerten Wasser,
   (iii) Zugabe einer starken anorganischen Säure, vorzugsweise Salzsäure, zu der gemäß Schritt (ii) erhaltenen Lösung oder Suspension oder zu dem in Schritt (i) bereitgestellten Wasser (hinsichtlich der alternativen Reihenfolge der Schritte siehe oben),
      so dass die wässrige Phase der angesäuerten Lösung oder Suspension einen pH-Wert von kleiner oder gleich 3 , vorzugsweise kleiner oder gleich 2 aufweist,
      so dass die bereitgestellte wässrige Lösung bzw. Suspension von Galactoarabinan 1 bis 35 Gew.-%, bevorzugt 5 bis 25 Gew.-%, Galactoarabinan enthält, bezogen auf das Gesamtgewicht der Lösung oder Suspension,
      und anschließend
   (iv) Erhitzen der bereitgestellten Lösung oder Suspension auf eine Temperatur im Bereich von 90 bis 125 °C und Halten bei dieser Temperatur für einen Zeitraum im Bereich von 10 bis 30 Stunden,
   (vi) Neutralisation der nach dem Erhitzen und dem Halten der Temperatur vorliegenden Lösung oder Suspension, vorzugsweise an einem oder nacheinander an mehreren Ionenaustauschern.
   (viii) Abtrennen der gebildeten D-Galactose gegebenenfalls im Gemisch mit weiteren Stoffen aus der resultierenden Produktmischung, vorzugsweise durch Kristallisation.

Wie bereits erwähnt, eignet sich ein erfindungsgemäß vorliegendes Verfahren besonders gut, um D-Galactose im industriellen Maßstab zu gewinnen bzw. bereitzustellen. Vorzugsweise wird das erfindungsgemäße Verfahren daher im industriellen Maßstab durchgeführt. "Industrieller Maßstab" bedeutet hierbei im Rahmen der vorliegenden Erfindung, dass eine gemäß einem erfindungsgemäßen Verfahren vorliegende Lösung oder Suspension von Galactoarabinan ein Volumen von zumindest 100 Litern besitzt.

In erfindungsgemäßen Verfahren zur Gewinnung von D-Galactose wird zum Ansäuern eine starke anorganische Säure eingesetzt. Wie bereits erwähnt, wird diese vorzugsweise zu einer gemäß Schritt (ii) erhaltenen Lösung bzw. Suspension oder zu einem in Schritt (i) bereitgestellten Wasser zugegeben, so dass die angesäuerte Lösung bzw. die wässrige Phase der Suspension einen pH-Wert von kleiner oder gleich 3, vorzugsweise kleiner oder gleich 2 aufweist. Für besonders bevorzugt einzustellende pH-Wert-Bereiche siehe unten. Als "starke" anorganische Säure gelten im Rahmen der vorliegenden Erfindung insbesondere anorganische Säuren, die einen pK_{A} von 2,2 oder weniger besitzen. Besonders bevorzugt ist die starke anorganische Säure ausgewählt aus der Gruppe bestehend aus: Salzsäure, Schwefelsäure, Phosphorsäure und deren Mischungen, wobei mit Salzsäure die besten Resultate erzielt wurden und diese daher in allen Gestaltungen des erfindungsgemäßen Verfahrens besonders bevorzugt ist.

Dementsprechend stellt eine besonders bevorzugte Ausführungsform der vorliegenden Erfindung ein erfindungsgemäßes Verfahren (wie oben beschrieben) dar, wobei zum Ansäuern eine starke anorganische Säure eingesetzt wird, die ausgewählt ist aus der Gruppe bestehend aus Salzsäure, Schwefelsäure, Phosphorsäure und deren Mischungen, vorzugsweise Salzsäure.

Gemäß einer besonders bevorzugten Ausgestaltung der vorliegenden Erfindung umfasst ein erfindungsgemäßes Verfahren (wie oben beschrieben) die folgenden Schritte:
- Bereitstellung der wässrigen Lösung oder Suspension von Galactoarabinan durch:
   (i) Bereitstellen von Wasser,
   (ii) Lösen oder Suspendieren von Galactoarabinan in dem in Schritt (i) bereitgestellten Wasser oder einem gemäß Schritt (iii) angesäuerten Wasser,
   (iii) Zugabe von Salzsäure (d.h. der generell bevorzugten Säure) zu der gemäß Schritt (ii) erhaltenen Lösung oder Suspension oder zu dem in Schritt (i) bereitgestellten Wasser,
      so dass die wässrige Phase der angesäuerten Lösung oder Suspension einen pH-Wert im Bereich von 1 bis 1,3 aufweist,
      so dass die bereitgestellte wässrige Lösung oder Suspension von Galactoarabinan 5 bis 25 Gew.-%, Galactoarabinan enthält, bezogen auf das Gesamtgewicht der Lösung oder Suspension,
   (iv) Erhitzen der bereitgestellten Lösung oder Suspension auf eine Temperatur im Bereich von 95 bis 110 °C und Halten bei dieser Temperatur für einen Zeitraum im Bereich von 20 bis 30 Stunden,
   (v) vorzugsweise Filtration (insbesondere bei Einsatz einer Suspension von Galactoarabinan),
   (vi) Neutralisation der nach dem Erhitzen und dem Halten der Temperatur bzw. nach der Filtration vorliegenden Lösung oder Suspension, vorzugsweise nacheinander zumindest an einem basischen und einem sauren Ionenaustauscher,
   (vii) vorzugsweise Verrühren der neutralisierten Mischung mit einer ethanolreichen Mischung von Ethanol und Wasser (Ansumpfen),
   (viii) Abtrennen der gebildeten D-Galactose gegebenenfalls im Gemisch mit weiteren Stoffen aus der resultierenden Produktmischung, vorzugsweise durch Kristallisation,
   (ix) gegebenenfalls Umkristallisation, vorzugsweise aus Wasser.

Eine Filtration und/oder ein Ansumpfen sind in erfindungsgemäßen Verfahren auch generell bevorzugt.

Vorzugsweise werden mehrere oder sämtliche der bevorzugten verfahrensgestaltenden Merkmale bzw. Parameter im vorliegenden erfindungsgemäßen Verfahren eingehalten. Bevorzugt umfasst demnach ein erfindungsgemäßes Verfahren sämtliche der beschriebenen Schritte (i), (ii), (iii), (iv), (v), (vi), (vii), (viii) und (ix), vorzugsweise jeweils in einer vorstehend als bevorzugt bezeichneten Ausgestaltung.

In eigenen Untersuchung wurden bei der Verwendung von Salzsäure zur Ansäuerung in einem erfindungsgemäßen Verfahren, wie bereits erwähnt, die besten Resultate erzielt. Die Verwendung von Salzsäure stellte sich für den gesamten Herstellprozess als besonders vorteilhaft heraus, insbesondere für die (saure) Hydrolyse des Galactoarabinans, aber auch zur Bereitstellung der wässrigen Lösung bzw. Suspension von Galactoarabinan (wie oben beschrieben). Zudem wirkte sich die Verwendung von Salzsäure vorteilhaft auf die in einem erfindungsgemäßen Verfahren vorzugsweise enthaltenen Schritte (iii), (iv), (vi), (vii) und (viii) aus. In weiterführenden Untersuchungen stellte sich heraus, dass sich das Gesamtverfahren bei der Verwendung von Schwefelsäure (37 gew.-%ig) anstelle von Salzsäure, insbesondere bei der Verwendung gemäß Schritt (iii) eines erfindungsgemäßen Verfahrens, insbesondere aber die Hydrolyse, das Ansumpfen und die Kristallisation, als deutlich schlechter bzw. schwieriger durchführbar erwiesen. Mit Salzsäure wurden die besten Ausbeuten erzielt.

In eigenen Untersuchung wurden bei der Verwendung von Salzsäure zur Ansäuerung gemäß einem erfindungsgemäß vorliegenden Verfahren, wie bereits erwähnt, die besten Resultate erzielt. Die Verwendung von Salzsäure stellte sich für den gesamten Herstellprozess als besonders vorteilhaft heraus, insbesondere für die (saure) Hydrolyse des Galactoarabinans, aber auch zur Bereitstellung der wässrigen Lösung bzw. Suspension von Galactoarabinan (wie oben beschrieben). Zudem wirkte sich die Verwendung von Salzsäure vorteilhaft auf die in einem erfindungsgemäßen Verfahren vorzugsweise enthaltenen Schritte (iii), (iv), (vi), (vii) und (viii) aus. In weiterführenden Untersuchungen stellte sich heraus, dass bei der Verwendung von Schwefelsäure (37 gew.-%ig) anstelle von Salzsäure, insbesondere bei der Verwendung gemäß Schritt (iii) eines bevorzugten Verfahrens, sich das Gesamtverfahren, insbesondere aber die Hydrolyse, das Ansumpfen und die Kristallisation, als deutlich schlechter bzw. schwieriger durchführbar erwiesen.

In der folgenden Tabelle werden vorteilhafte Effekte beschrieben, die im Rahmen eigener Untersuchungen zur näheren Charakterisierung einiger vorzugsweise in einem erfindungsgemäßen Verfahren enthaltenen Verfahrensschritte beobachtet werden konnten. Die im Folgenden als vorteilhaft beschriebenen Parameter und Merkmale der Reaktionsführung sind für ein erfindungsgemäßes Verfahren besonders bevorzugt, unabhängig voneinander und - vorzugsweise - in Kombination miteinander.

| Verfahrensschritt | Effekt |
|---|---|
| Hydrolyse | Verwendung von Salzsäure besonders vorteilhaft, mit Schwefelsäure verläuft die Hydrolyse unvollständiger und unsauberer. |
| | |
| | pH = 1-1,3 bevorzugt, da bei höherem pH-Wert die Reaktion nach 24 Std. noch unvollständig ist; in noch stärker saurem Milieu findet Abbau der D-Galactose statt. |
| | |
| | Die angegebene Verdünnung der Säure ist vorteilhaft, da bei höherer Konzentration die Hydrolyse nicht vollständig verläuft. |
| | |
| Filtration | Eine Filtration hält die unlöslichen Bestandteile zurück, die sonst eine gegebenenfalls durchgeführte Kristallisation behindern können. |
| | |
| Neutralisation | Ohne Neutralisation kann der pH-Wert bei einem gegebenenfalls stattfindenden Eindampfen zu weit absinken, so dass die D-Galactose (teilweise) zerstört würde. |
| | |
| | Vorteilhafterweise wird bei einer Neutralisation über Ionenaustauscher auch gleichzeitig eine Filtration erreicht. |
| | |
| Ansumpfen (Verrühren mit einer ethanolreichen Mischung aus Ethanol und Wasser) | Dadurch werden Nebenbestandteile wie Arabinose, Galacturonsäure und/oder Polymerreste zum Teil herausgelöst. Vorzugsweise nachfolgende Kristallisationen verlaufen erfolgreicher, da das Ausgangsmaterial reiner vorliegt. |
| | |
| Eindampfen | Eine Reduktion der Wassermenge ist vorteilhaft, damit die Rohware leichter zur Kristallisation gebracht werden kann. |
| | |
| Umkristallisation | Hierdurch wird eine Aufreinigung des Produktes zu 98% iger Ware erreicht, die Kristallisation erfolgt vorzugsweise aus Wasser, wobei die Kristallisation der D-Galactose vorzugsweise durch Zugabe von Ethanol initiiert wird. |

Dementsprechend umfasst ein bevorzugtes erfindungsgemäßes Verfahren vorzugsweise den weiteren Schritt:
- Reduktion der Wassermenge (vorzugsweise destillative Einengung) in der (gegebenenfalls filtrierten und gegebenenfalls neutralisierten) nach dem Erhitzen und dem Halten der Temperatur vorliegenden Lösung bzw. Suspension (Eindampfen).

Als besonders bevorzugter Schritt in einem erfindungsgemäßen Verfahren hat sich das Ansumpfen herausgestellt. Unter Ansumpfen wird das Herauslösen von Nebenbestandteilen aus der rohen D-Galactose durch Verrühren mit einer ethanolreichen Mischung von Ethanol und Wasser verstanden, wobei in diesen Mischungen der Ethanolanteil vorzugsweise im Bereich von 75 - 98 Gew.-%, weiter bevorzugt im Bereich von 80 - 95 Gew.-% liegt. Durch das Ansumpfen wird eine Reinheit von D-Galactose nach Kristallisation von 98% oder mehr erreicht.

Die nach dem erfindungsgemäßen Verfahren erhaltene D-Galactose weist eine hohe Reinheit auf, ist gut kristallisierbar und weist ein charakteristisches Nebenproduktspektrum auf (Fingerprint).

Ein weiterer Aspekt der vorliegenden Erfindung betrifft somit eine Mischung umfassend D-Galactose, herstellbar nach einem erfindungsgemäßen Verfahren (wie oben beschrieben), vorzugsweise in einer vorstehend als bevorzugt bezeichneten Ausgestaltung.

Ein weiterer Aspekt der vorliegenden Erfindung betrifft eine Mischung umfassend D-Galactose (vorzugsweise wie oben beschrieben), die folgende Bestandteile umfasst oder aus ihnen besteht:
Galacturonsäure: bis zu 1 Gew.-%, vorzugsweise 0,2 - 1 Gew.-%,
L-Arabinose: bis zu 1,5 Gew.-%, vorzugsweise 0,3 - 1,5 Gew.-%,
D-Galactose: 97,5 - 99,5 Gew.-%. Die Gewichtsprozentangaben beziehen sich dabei jeweils auf die Gesamttrockenmasse der Mischung.
Vorzugsweise besitzt eine wie oben beschriebene erfindungsgemäße Mischung umfassend die D-Galactose eine Restfeuchte von höchstens 0,5 Gew.-%, bevorzugt von höchstens 0,3 Gew.-%.

Für die Zwecke der vorliegenden Erfindung und die zu lösende Aufgabe wird in dem erfindungsgemäßen Verfahren Galactoarabinose aus pflanzlichem Ausgangsmaterial eingesetzt. Dementsprechend ist eine erfindungsgemäße, D-Galactose umfassende Mischung vorzugsweise frei von Lactose.

Ein erfindungsgemäßes Verfahren (wie oben beschrieben) zur Gewinnung von D-Galactose bzw. zur Herstellung einer erfindungsgemäßen Mischung umfassend D-Galactose (wie oben beschrieben) wird in einem bevorzugten Beispiel wie folgt durchgeführt:

In 4000-5000 L Wasser werden 500 kg Galactoarabinan (z.B. FiberAid^{®}) gelöst bzw. suspendiert. Es wird mit ca. 50-60 L Salzsäure (30 gew.-%ig) auf einen pH-Wert im Bereich von 1,0-1,5 eingestellt. Die Temperatur wird auf 100°C eingestellt. Die Hydrolyse läuft 24 Stunden (Hydrolysekontrolle mittels HPLC). Nach beendeter Hydrolyse wird auf 50-60 °C abgekühlt, filtriert (beispielsweise mittels einer Kammerfilterpresse, beispielsweise mit Filtrierhilfsmittel: Hyflo) und neutralisiert, indem die Lösung bzw. die Suspension über einen alkalischen Ionenaustauscher, anschließend über einen sauren Ionenaustauscher und dann noch einmal über einen alkalischen Ionentauscher gegeben wird. Die Ionentauscher werden mit ca. 2000 L Stadtwasser gespült. Dann wird die Lösung bzw. die Suspension destillativ eingeengt (auf ca. 1500 L) und zur Kristallisation gebracht. Die Rohware wird abgeschleudert (Zentrifuge). Die Rohware wird mit 500 L 90 gew.-%igem Ethanol (Rest: Wasser) versetzt und bei einer Temperatur von ca. 20°C eine Stunde gerührt. Durch Filtration (beispielsweise eine Kammerfilterpresse) wird die Ansumpflauge vom Feststoff getrennt. Dieser Vorgang wird zweimal durchgeführt. Die D-Galactose-Rohware wird zum Umkristallisieren gelöst (100°C, Wasser, gesättigte Lösung), mit Aktivkohle versetzt und beispielsweise über das Filtrierhilfsmittel Hyflo filtriert. Aus dem Filtrat kann die D-Galactose erneut kristallisiert und anschließend abzentrifugiert werden. Gegebenenfalls kann für eine weitere Aufreinigung erneut umkristallisiert werden (gesättigte Lösungs-Kristallisation). Abschließend wird die so erhaltene D-Galactose getrocknet (beispielsweise Umluft- oder Vakuumtrockenschrank) und auf die gewünschte Partikelgröße vermahlen.

Weitere Aspekte der vorliegenden Erfindung ergeben sich aus den Beispielen und den beigefügten Patentansprüchen.

Nachfolgend wird die Erfindung anhand von Beispielen näher erläutert. Sofern nicht anders angegeben, beziehen sich alle Angaben auf das Gewicht.

### Beispiel: Gewinnung von D-Galactose aus Galactoarabinan

55 kg Salzsäure (30 gew.-%ig) werden in 4500 L Wasser (städtisch) vorgelegt und auf 80°C erwärmt. In die dabei entstandene saure Lösung werden 500 kg Galactoarabinan (Lara Care A^{®} 200) unter Rühren eingebracht und die erhaltene Suspension bzw. Lösung wird auf 100°C erhitzt. Nach 24 Stunden Reaktionszeit bei 100°C wird die Reaktionsmischung auf ca. 60°C abgekühlt und filtiert. Anschließend wird die Reaktionsmischung eingedampft und eine erste Kristallisation durchgeführt. Nach dem Abzentrifugieren der Kristalle werden diese erforderlichenfalls ein- oder mehrmals aus Wasser umkristallisiert, abzentrifugiert, getrocknet und auf die gewünschte Partikelgröße gemahlen.

Der getrocknete und homogenisierte Feststoff wurde per HPLC analysiert. Typischerweise wurde eine Reinheit von größer oder gleich 97% erreicht.

HPLC-Chromatographiebedingungen:
Säule: Varian MetaCarb 87C
Temperatur: 75°C
Fluss: 0,5 L/min
Eluent: HPLC-Wasser
Detektion: Brechungsindex
Galacturonsäure (Retentioszeit 10-12 min.): bis zu 1 Gew.-%
L-Arabinose (Retentioszeit 15-17 min.): bis zu 1,5 Gew.-%
D-Galactose (Retentioszeit 13-15 min.): 97,5 - 99 Gew.-%

## Patentansprüche

1. Verfahren zur Gewinnung von D-Galactose, mit folgenden Schritten:
- Bereitstellen einer wässrigen Lösung oder Suspension von Galactoarabinan, wobei die wässrige Lösung bzw. Phase einen pH-Wert von kleiner oder gleich 3 aufweist,
- Erhitzen der Lösung bzw. Suspension auf eine Temperatur im Bereich von 80 bis 160 °C und Halten bei dieser Temperatur für einen Zeitraum im Bereich von 1 bis 40 Stunden,
- Abtrennen der gebildeten D-Galactose gegebenenfalls im Gemisch mit weiteren Stoffen aus der resultierenden Produktmischung.

2. Verfahren nach Anspruch 1, mit folgenden Schritten
- Bereitstellen der wässrigen Lösung oder Suspension von Galactoarabinan durch:
(i) Bereitstellen von Wasser,
(ii) Lösen oder Suspendieren von Galactoarabinan in dem in Schritt (i) bereitgestellten Wasser oder einem gemäß Schritt (iii) angesäuerten Wasser,
(iii) Zugabe einer starken anorganischen Säure zu der gemäß Schritt (ii) erhaltenen Lösung oder Suspension oder zu dem in Schritt (i) bereitgestellten Wasser, so dass die wässrige Phase der angesäuerten Lösung oder Suspension einen pH-Wert von kleiner oder gleich 3, vorzugsweise kleiner oder gleich 2 aufweist.

3. Verfahren nach Anspruch 1 oder 2, wobei das Galactoarabinan ein mittleres Molekulargewicht im Bereich von 2000 - 250000 Dalton aufweist, bevorzugt ein mittleres Molekulargewicht im Bereich von 5000 - 100000 Dalton und besonders bevorzugt ein mittleres Molekulargewicht im Bereich von 5000 - 50000 Dalton.

4. Verfahren nach einem der vorangehenden Ansprüche, wobei die bereitgestellte wässrige Lösung bzw. Suspension von Galactoarabinan 1 bis 35 Gew.-%, bevorzugt 5 bis 25 Gew.-%, Galactoarabinan enthält, bezogen auf das Gesamtgewicht der Lösung bzw. Suspension.

5. Verfahren nach einem der vorangehenden Ansprüche, mit folgendem Schritt:
- Erhitzen der Lösung bzw. Suspension auf eine Temperatur im Bereich von 90 bis 125 °C und Halten bei dieser Temperatur für einen Zeitraum im Bereich von 10 bis 30 Stunden.

6. Verfahren nach einem der vorangehenden Ansprüche, mit folgendem Schritt:
- Neutralisation der nach dem Erhitzen und dem Halten der Temperatur vorliegenden Lösung oder Suspension, vorzugsweise an einem oder nacheinander an mehreren Ionenaustauschern.

7. Verfahren nach einem der vorangehenden Ansprüche, mit folgendem Schritt:
- Abtrennen der gebildeten D-Galactose, gegebenenfalls im Gemisch mit weiteren Stoffen, aus der resultierenden Produktmischung durch Kristallisation.

8. Verfahren nach einem der vorangehenden Ansprüche, mit folgenden Schritten:
- Bereitstellung der wässrigen Lösung oder Suspension von Galactoarabinan durch:
(i) Bereitstellen von Wasser,
(ii) Lösen oder Suspendieren von Galactoarabinan in dem in Schritt (i) bereitgestellten Wasser oder einem gemäß Schritt (iii) angesäuerten Wasser,
(iii) Zugabe einer starken anorganischen Säure, vorzugsweise Salzsäure, zu der gemäß Schritt (ii) erhaltenen Lösung bzw. Suspension oder zu dem in Schritt (i) bereitgestellten Wasser,
so dass die wässrige Phase der angesäuerten Lösung oder Suspension einen pH-Wert von kleiner oder gleich 3 , vorzugsweise kleiner oder gleich 2 aufweist,
so dass die bereitgestellte wässrige Lösung oder Suspension von Galactoarabinan 1 bis 35 Gew.-%, bevorzugt 5 bis 25 Gew.-%, Galactoarabinan enthält anschließend, bezogen auf das Gesamtgewicht der Lösung oder Suspension,
und anschließend:
(iv) Erhitzen der bereitgestellten Lösung oder Suspension auf eine Temperatur im Bereich von 90 bis 125 °C und Halten bei dieser Temperatur für einen Zeitraum im Bereich von 10 bis 30 Stunden,
(vi) Neutralisation der nach dem Erhitzen und dem Halten der Temperatur vorliegenden Lösung oder Suspension, vorzugsweise an einem oder nacheinander an mehreren Ionenaustauschern.
(viii) Abtrennen der gebildeten D-Galactose gegebenenfalls im Gemisch mit weiteren Stoffen aus der resultierenden Produktmischung, vorzugsweise durch Kristallisation.

9. Verfahren nach einem der vorangehenden Ansprüche, wobei zum Ansäuern eine starke anorganische Säure eingesetzt wird, die ausgewählt ist aus der Gruppe bestehend aus Salzsäure, Schwefelsäure, Phosphorsäure und deren Mischungen, vorzugsweise Salzsäure.

10. Verfahren nach einem der vorangehenden Ansprüche, mit folgenden Schritten:
- Bereitstellung der wässrigen Lösung oder Suspension von Galactoarabinan durch:
(i) Bereitstellen von Wasser,
(ii) Lösen oder Suspendieren von Galactoarabinan in dem in Schritt (i) bereitgestellten Wasser oder einem gemäß Schritt (iii) angesäuerten Wasser,
(iii) Zugabe von Salzsäure, zu der gemäß Schritt (ii) erhaltenen Lösung oder Suspension oder zu dem in Schritt (i) bereitgestellten Wasser, so dass die wässrige Phase der angesäuerten Lösung oder Suspension einen pH-Wert im Bereich von 1 bis 1,3 aufweist,
so dass die bereitgestellte wässrige Lösung oder Suspension von Galactoarabinan 5 bis 25 Gew.-%, Galactoarabinan enthält, bezogen auf das Gesamtgewicht der Lösung oder Suspension,
und anschließend:
(iv) Erhitzen der bereitgestellten Lösung oder Suspension auf eine Temperatur im Bereich von 95 bis 110 °C und Halten bei dieser Temperatur für einen Zeitraum im Bereich von 20 bis 30 Stunden,
(v) vorzugsweise Filtration,
(vi) Neutralisation der nach dem Erhitzen und dem Halten der Temperatur bzw. nach der Filtration vorliegenden Lösung oder Suspension, vorzugsweise nacheinander zumindest an einem basischen und einem sauren Ionenaustauscher,
(vii) vorzugsweise Verrühren der neutralisierten Mischung mit einer ethanolreichen Mischung von Ethanol und Wasser (Ansumpfen),
(viii) Abtrennen der gebildeten D-Galactose gegebenenfalls im Gemisch mit weiteren Stoffen aus der resultierenden Produktmischung, vorzugsweise durch Kristallisation,
(ix) gegebenenfalls Umkristallisation, vorzugsweise aus Wasser.

11. Mischung umfassend D-Galactose, herstellbar nach einem Verfahren gemäß einem der vorangehenden Ansprüche.

12. Mischung umfassend D-Galactose, vorzugsweise nach Anspruch 11, umfassend oder bestehend aus:
Galacturonsäure: bis zu 1 Gew.-%, vorzugsweise 0,2 - 1 Gew.-%,
L-Arabinose: bis zu 1,5 Gew.-%, vorzugsweise 0,3 - 1,5 Gew.-%,
D-Galactose: 97,5 - 99,5 Gew.-%,
wobei sich die Gewichtsprozentangaben auf die Gesamttrockenmasse der Mischung beziehen.

13. Mischung nach Anspruch 11 oder 12, mit einer Restfeuchte von höchstens 0,5 Gew.-%, bevorzugt von höchstens 0,3 Gew.-%.
